# EUROPÄISCHE PATENTANMELDUNG

(11) **EP 1 767 194 A1**
(43) Veröffentlichungstag der Anmeldung: **28.03.2007**
(21) Anmeldenummer: 05012467.6
(22) Anmeldetag: 09.06.2005
(51) Int. Cl.: A61K 9/19, A61K 9/14, A61K 9/20, A61K 31/585, A61K 31/567

(54) **Verfahren zur Herstellung von Adsorbaten des Drospirenons**

(71) Anmelder: Helm AG, 20097 Hamburg (DE)
(72) Erfinder: Glänzer, Klaus, Dr., 22337 Hamburg (DE)
(74) Vertreter: Becker Kurig Straus

(57) **Zusammenfassung**

Die vorliegende Erfindung betrifft ein neuartiges Verfahren zur Herstellung von Adsorbaten des Drospirenons. Die vorliegende Erfindung betrifft ferner pharmazeutische Zubereitungen, diese Drospirenon-Adsorbate in morphologisch stabiler Form enthaltend. Diese pulverförmigen Zubereitungen sind insbesondere dadurch charakterisiert, dass sie den in wässerigen Phasen schwer löslichen Wirkstoff so freisetzen, dass das Dissolutionsprofil daraus hergestellter pharmazeutischer Präparate den Anforderungen für einen Einsatz als Kontrazeptivum entspricht. Die erfindungsgemäßen pulverförmigen Zubereitungen können auch in Kombination mit anderen pharmazeutischen Wirkstoffen, z.B. mit Gestagenen und Estrogenen, auf einfache Weise zur Herstellung von Granulaten und von durch Direktverpressung hergestellten Tabletten verwendet werden.

## Beschreibung

Die vorliegende Erfindung betrifft ein neuartiges Verfahren zur Herstellung von Adsorbaten des Drospirenons. Die vorliegende Erfindung betrifft ferner pharmazeutische Zubereitungen, die diese Drospirenon-Adsorbate in morphologisch stabiler Form enthalten. Diese pulverförmigen Zubereitungen sind insbesondere dadurch charakterisiert, dass sie den in wässerigen Phasen schwer löslichen Wirkstoff so freisetzen, dass das Dissolutionsprofil daraus hergestellter pharmazeutischer Präparate den Anforderungen für einen Einsatz als Kontrazeptivum entspricht. Die erfindungsgemäßen pulverförmigen Zubereitungen können auch in Kombination mit anderen pharmazeutischen Wirkstoffen, z.B. mit Gestagenen und Estrogenen, auf einfache Weise zur Herstellung von Granulaten und von durch Direktverpressung hergestellten Tabletten verwendet werden.

Drospirenon (INN) ist ein synthetisches Gestagen, das zudem Aldosteron-antagonistische und antiandrogene Eigenschaften besitzt. Die seit 1976 bekannte Substanz mit der chemischen Bezeichnung 6β, 7β; 15β, 16β-Dimethylen-3-oxo-17α-pregn-4-en-21, 17-carbolacton wird als orales Kontrazeptivum, z.B. in Kombination mit Estradiol oder Ethinylestradiol eingesetzt (siehe EP 1 214 076 B1).

Drospirenon ist gering löslich in Wasser oder wässerigen Pufferlösungen, so dass es ohne weitere Maßnahmen nur zu einem geringen Teil bioverfügbar ist. In diesem Zusammenhang ist bekannt, dass im Allgemeinen die Auflösegeschwindigkeit von Steroid-Wirkstoffen von der Partikelgröße abhängig ist und durch Mikronisierung verbessert werden kann ("Bioavailability of Orally Administered Sex Steroids Used in Oral Contraception and Hormone Replacement Therapy", K. Fotherby, Contraception 1996, 54, 59-69).

Um eine für den Einsatz als Kontrazeptivum geeignete Freisetzung zu erzielen, muss Drospirenon daher aufwendig vorbereitet werden. Gemäß EP 1 214 076 B1 wird ein mikronisierter Wirkstoff mit einer Oberfläche > 10.000 cm²/g eingesetzt, um eine Freisetzung von mind. 70 % Wirkstoff aus einer 3 mg Drospirenon enthaltenden Tablette innerhalb von 30 Min. zu erhalten. Die genannte Oberfläche kann auch dadurch erzielt werden, dass eine Drospirenon-Lösung (z.B. Ethylacetat als Lösungsmittel) auf einen inerten Träger gesprüht wird.

Mikronisierungen, Vermahlungen oder Versprühungen von hochaktiven hormonartigen Stoffen wie Drospirenon müssen unter besonderen, ihre hohe Toxizität berücksichtigenden Bedingungen (Staubschmutz, Luftfiltration, usw.) durchgeführt werden. Sie sind daher technisch sehr aufwendig und führen zudem zu Substanzverlusten, die bei diesen Stoffen, neben der Umwelttoxizität, erhebliche wirtschaftliche Nachteile darstellen.

Drospirenon ist darüber hinaus eine instabile Substanz, die durch sauren Einfluss in einer Isomerisierungsreaktion zu einem physiologisch inaktiven Stoff umgewandelt, bzw. durch alkalischen Einfluss hydrolytisch abgebaut werden kann. Solche Einflüsse können insbesondere bei allen Feuchtgranulationsprozessen oder beim Versprühen von Drospirenon-Lösungen zu Instabilität, bzw. Abbau führen.

Daher ist es Aufgabe der vorliegenden Erfindung, ein einfaches und kostengünstiges Verfahren zur Herstellung oraler Drospirenon-haltiger Formulierungen zu entwickeln, die die oben beschriebenen Nachteile vermeiden.

Weiterhin ist es Aufgabe dieser Erfindung, Wirkstoff-haltige Pulver zu entwickeln, die Drospirenon in morphologisch stabiler Form enthalten.

Weiterhin ist es Aufgabe dieser Erfindung, ein Herstellungsverfahren zur Gewinnung dieser Wirkstoff-haltigen Pulver, die Drospirenon in morphologisch stabiler Form enthalten, zu entwickeln.

Weiterhin ist es Aufgabe dieser Erfindung, ein Verfahren zur Herstellung oraler pharmazeutischer Formulierungen in Form von Tabletten und Granulaten zu entwickeln, die auf den oben genannten Drospirenon-haltigen Pulvern basieren, und deren Freisetzungsprofil den Anforderungen für einen Einsatz als Kontrazeptivum entspricht.

Diese Aufgaben werden erfindungsgemäß durch ein Verfahren zur Herstellung von Wirkstoffpulvern, enthaltend Drospirenon, gemäß Anspruch 1 gelöst. Dabei geht man von einer Lösung des Drospirenons in einem vorwiegend organischen Lösungsmittel aus, suspendiert und/oder löst darin ein pharmazeutisch akzeptables Adsorbermaterial und entfernt anschließend das Lösungsmittel auf geeignete Weise, insbesondere durch Trocknen. Der Gesamtwassergehalt des organischen Lösungsmittels beträgt erfindungsgemäß nicht mehr als 15 Vol.-%, bevorzugt nicht mehr als 5 Vol.-% (vergleiche Anspruch 1).

Die obigen Aufgaben werden weiterhin durch die Drospirenon-haltigen Wirkstoffpulver gemäß den Ansprüchen 6 und 7, die pharmazeutische Formulierung gemäß Anspruch 8 sowie durch die Verwendung gemäß Anspruch 12 gelöst.

In den Unteransprüchen werden vorteilhafte Ausführungsformen der Erfindung beschrieben.

Als erfindungsgemäße Adsorbermaterialien werden Polymerisate auf Basis von Acryl- und/oder Methacrylsäure oder deren Aminoalkylestern oder deren Ammonioalkylestern und/oder deren Alkylestern eingesetzt. Typische erfindungsgemäße Polymerisate sind daher Homo- oder Copolymerisate von Acrylmonomeren. Sie können neutral sein und ganz oder nahezu ganz aus Alkylestern der Acryl- und/oder Methacrylsäure aufgebaut sein. Polymerisate, die in Wasser oder in den sauren oder schwach alkalischen Flüssigkeiten des Magen-Darm-Traktes löslich oder quellbar sind, enthalten Monomereinheiten mit polaren Gruppen, meist als Comonomer-Einheiten neben unpolaren Einheiten, wie den Alkylestern der Acryl- und/oder Methacrylsäure.

Die erfindungsgemäß eingesetzten Polymerisate sind daher vorzugsweise zu 30 bis 70 Gew.-% aus Acryl- und/oder Methacrylsäure, oder bis zu 5 bis 50 Gew.-% aus deren Aminoalkylestern oder ganz oder zum restlichen Teil aus Alkylestern der Acryl- und/oder Methacrylsäure aufgebaut. Gegebenenfalls können bis zu 50 Gew.-% an anderen, damit radikalisch copolymerisierbaren ethylenisch ungesättigten Monomeren am Aufbau der Polymerisate beteiligt sein. Unter den Alkylestern sind diejenigen mit 1 bis 4 Kohlenstoffatomen im Alkylrest bevorzugt, wozu noch Alkylsubstituenten mit 1 bis 4 Kohlenstoffatomen am Aminrest von Aminoalkylestern kommen können. Aus pharmakologischer Sicht sind Ethylacrylat und Methylmethacrylat die bestgeeigneten Esterkomponenten.

Bevorzugte Adsorbermaterialien sind daher erfindungsgemäß Polymethacrylate, die z. B. als Eudragit® kommerziell erhältlich sind (siehe hierzu Fiedler, Lexikon der Hilfsstoffe, Editio Cantor, [2002], S. 687-690), besonders bevorzugte Trägermaterialien sind Aminoalkylmethacrylate.

Das erfindungsgemäße Verfahren bezieht sich auf die Herstellung von Drospirenon-haltigen Wirkstoffpulvern, welche als ersten Wirkstoff Drospirenon, d.h. 6β, 7β; 15β, 16β-Dimethylen-3-oxo-17α-pregn-4-en-21, 17-carbolacton enthalten.

Die pharmazeutische Formulierung kann eine entsprechende Tagesdosis von etwa 2 mg bis etwa 4 mg von Drospirenon enthalten. Falls in der pharmazeutischen Formulierung wenigstens ein weiterer Wirkstoff aus der Gruppe der Estrogene, wie z. B. Estron, Estriol, Mestranol, Estradiol, Ethinylestradiol und dergleichen enthalten ist, kann dieser in einer Tagesdosis von etwa 0,01 mg bis etwa 0,05 mg, gegebenenfalls zusammen mit einem oder mehreren pharmazeutisch akzeptablen Hilfsstoffen, vorhanden sein.

In diesem Zusammenhang soll darauf hingewiesen werden, dass abgesehen von den aktiven Wirkstoffen selbst, auch Ester oder Prodrugs von Drospirenon im Sinne der vorliegenden Erfindung eingesetzt werden können, z.B. ein Oxyiminopregnancarbolacton, wie dies z.B. in WO 98/24801 beschrieben ist. In ähnlicher Weise können Ester oder Ether der Estrogenkomponente (Estron, Estriol, Mestranol, Estradiol, Ethinylestradiol) ebenfalls in den erfindungsgemäßen pharmazeutischen Zubereitungen enthalten sein.

Erfindungsgemäß wird ein Verhältnis von Wirkstoff, das heißt, von Drospirenon, zu Adsorbermaterial (Acrylpolymer) im Bereich von 1:0,05 bis 1:20, insbesondere im Bereich von 1:1 bis 1:10, eingestellt.

Die Erfindung betrifft weiterhin pharmazeutische Formulierungen, enthaltend diese neuen Wirkstoff-haltigen Pulver mit Drospirenon-Adsorbaten in morphologisch stabiler Form. Die erfindungsgemäßen pharmazeutischen Formulierungen enthalten gegebenenfalls weitere Hilfsstoffe und können in die gewünschte Darreichungsform überführt werden. Besonders bevorzugt sind den Wirkstoff schnell freisetzende, durch Direktverpressung hergestellte Tabletten, die gegebenenfalls beschichtet werden können.

Für das erfindungsgemäße Verfahren zur Herstellung der Wirkstoff-haltigen Pulvern, die Drospirenon in morphologisch stabiler Form enthalten, eignen sich organische Lösungsmittel für die den pharmazeutischen Wirkstoff enthaltende Lösung. Die organischen Lösungsmittel sind insbesondere ausgewählt aus der Gruppe der niederen Alkanole mit 1 bis 4 Kohlenstoffatomen, der Gruppe der Ether, der Ester, der Gruppe der mehrwertigen Alkohole wie z.B. Glykol, und der Gruppe der aliphatischen Ketone und Gemische der vorgenannten Lösungsmittel. Besonders bevorzugt sind Methanol, Ethanol, Isopropanol, n-Propanol, Aceton und andere Lösungsmittel wie Ethylacetat, Methylethylketon, Methyl-t.butylester (MTBE), Acetonitril, Tetrahydrofuran, Diethylether, sowie Gemische der vorgenannten Lösungsmittel. Erfindungsgemäß wird bevorzugt unter Inertgasatmosphäre, wie z. B. unter Stickstoffatmosphäre gearbeitet und, falls dies notwendig ist, werden entgaste bzw. sauerstofffreie Lösungen verwendet. Die Lösungen können auch andere Arzneistoffe oder Zusätze pH-puffernder Stoffe enthalten.

Für die Herstellung der pharmazeutischen Formulierungen, wobei insbesondere Tabletten bevorzugt sind, können alle üblichen pharmazeutischen Hilfsstoffe verwendet werden. Als Füllstoffe und/oder Bindemittel können z.B. Cellulosen bzw. Cellulosederivate (z. B. mikrokristalline Cellulose, native Cellulose, Hydroxypropylcellulose, Hydroxypropylmethylcellulose, Methylcellulose), Zucker (z.B. Lactose, Fructose, Saccharose, Glucose, Maltose), Zuckeralkohole (z.B. Lactit, Mannit, Sorbit, Xylit), anorganische Füllstoffe (z.B. Calciumphosphate und Calciumsulfate) und Stärken (z.B. Maisstärke, Kartoffelstärke, Weizenstärke, Dextrine, pregelatinisierte Stärken) verwendet werden. Darüber hinaus können alle weiteren Hilfsstoffe, die dem Fachmann aufgrund seines galenischen Basiswissens bekannt sind, wie z.B. Schmiermittel, Zerfallhilfsmittel, Netzmittel, Stoffe zur Verbesserung des Fliessverhaltens, alkalische oder saure Zusatzstoffe, Stabilisatoren, Aromen, Farbpigmente und Farbstoffe zur Herstellung der erfindungsgemäßen Arzneiformulierungen eingesetzt werden.

Bei der Herstellung von Kombinationspräparaten z.B. mit Estrogenen (Estron, Estriol, Mestranol, Estradiol, Ethinylestradiol, usw.), können die entsprechenden Stoffe, sofern sie nicht bereits in gelöster Form in den zur Herstellung der erfindungsgemäßen Drospirenon-Lösungen enthalten sind, auf bekannte Weise verarbeitet werden.

Der Anteil an Bindemitteln in der Gesamtmischung der Arzneimittelzubereitung ist bevorzugt 0 bis 20 Gew.-%, der Anteil an Füll- und Hilfsstoffen in der Gesamtmischung beträgt 20 bis 99 Gew.-%, bevorzugt von 50 bis 99 Gew.-%.

Mit dem erfindungsgemäßen Verfahren werden lagerstabile Drospirenon-Zubereitungen hergestellt, die zudem durch eine schnelle Wirkstofffreisetzung in Wasser unter physiologischen Bedingungen charakterisiert sind. Es wurde erfindungsgemäß ein Verfahren gefunden, das ausgehend von einer Lösung des Drospirenons in einem organischen Lösungsmittel zu direkt weiterverarbeitbarem Pulver führt.

Die Drospirenon-Wirkstofflösung kann grundsätzlich in einer Ausführungsform der Erfindung durch Auflösen des Drospirenons in einem geeigneten organischen Lösungsmittel hergestellt werden. Andere wirksame Stoffe einer Arzneimittelkombination können, sofern ihre Löslichkeit in dem gewählten Lösungsmittel dies gestattet, grundsätzlich auf gleiche Weise mit gelöst und zu den Drospirenon-haltigen Pulvern verarbeitet werden.

Der Trocknungsvorgang kann durch Temperieren und Vakuum unterstützt werden. Gleichermaßen kann der Trocknungsvorgang als Gefriertrocknung erfolgen. Das Lösungsmittel kann durch Arbeiten im geschlossenen System zurückgewonnen und für den Folgeprozess wieder eingesetzt werden. Gemäß dem beschriebenen erfindungsgemäßen Verfahren hergestellte Drospirenon-haltige Zubereitungen können direkt zur Weiterverarbeitung zu Arzneiformen wie Tabletten oder Granulaten eingesetzt werden, bevorzugt zur Weiterverarbeitung mittels eines Direktkompressionsverfahrens.

Gegebenenfalls können die so erhaltenen Arzneiformen für spezielle Anwendungen mit den üblichen Filmüberzügen, z.B. zur Freisetzungssteuerung und/oder zur Geschmacksmaskierung und/oder zur Verbesserung der Stabilität versehen werden, wie dies z.B. in Kapitel 9 der Monographie von W. A. Ritschel, Die Tablette, Verlag Editio Cantro [2002] und in den dort zitierten Literaturstellen beschrieben wird.

Überraschenderweise wurde gefunden, dass nach dem erfindungsgemäßen Verfahren hergestellte Zubereitungen den Wirkstoff Drospirenon in der für Arzneimittel erforderlichen homogenen Verteilung aufweisen und diesen schnell und uneingeschränkt freisetzen.

Die genannten Eigenschaften bleiben auch insbesondere dann erhalten, wenn diese Drospirenon-Zubereitungen zu Arzneiformen, wie z.B. Tabletten verarbeitet werden.

Die Erfindung wird nun anhand der folgenden Beispiele und Figuren näher erläutert, ohne jedoch die Erfindung darauf zu beschränken.

### Beispiele 1 bis 3

### Beispiel 1: Herstellung von Tabletten mit Drospirenon, Rezeptur 1 (erfinduneseemäß)

Zu einer Lösung von 1g Drospirenon in 16 ml Aceton werden 2g Polymethacrylat (Eudragit® E) gegeben und unter leichtem Erwärmen (30 bis 40°C) gelöst. Die klare Lösung wird gefriergetrocknet, so dass ein feines Wirkstoff-haltiges Pulver entsteht. Das Pulver wird mit pharmazeutischen Hilfsstoffen gemäß folgender Rezeptur vermischt und zu Tabletten verpresst:
■ Drospirenon-Pulver, entsprechend 2,5 mg Drospirenon 7,5 mg
■ Mikrokristalline Cellulose (Celphere SCP-100®) 77,5 mg
■ Hilfsstoffe (Croscarmellose-Natrium, Natriumlaurylsulfat,
   Siliziumdioxid, Magnesiumstearat) in den üblichen Mengen 15 mg

Die verwendeten Mengen der weiteren Hilfsstoffe sind dem Fachmann aufgrund seines Basiswissens bekannt und können Standardwerken zur Formulierung von Tabletten, wie z.B. Ritschel et al., die Tablette, Kapitel 2, Editio Cantor - Aulendorf, 2. Auflage [2002] entnommen werden.

Eigenschaften der pressfertigen Mischung und der Tabletten:
■ Kompressibilität und Fließfähigkeit: gut
■ Mittlere Härte: 88 N
■ Abrieb: < 0, 1 % (bestimmt nach Ph. Eur.)
■ Zerfallzeit: 70 Sek. (bestimmt nach Ph. Eur.)
■ Freisetzung: 82% nach 15 Min.

Die so erhaltenen Tabletten können gegebenenfalls mit einem Überzug versehen werden.

### Beispiel 2: Herstellung von Tabletten mit Drospirenon, Rezeptur 2 (erfindungsgemäß)

Zu einer Lösung von 1g Drospirenon und 0,01 g Ethinylestradiol in 20 ml Aceton werden 2 g Polymethacrylat (Eudragit® E) gegeben und unter leichtem Erwärmen (30 bis 40°C) gelöst. Die klare Lösung wird gefriergetrocknet, so dass ein feines Wirkstoff-haltiges Pulver entsteht. Das Pulver wird mit pharmazeutischen Hilfsstoffen gemäß folgender Rezeptur vermischt und zu Tabletten verpresst:
■ Drospirenon-Pulver, entsprechend 4,0 mg Drospirenon
   und 0,04 mg Ethinylestradiol 12 mg
■ Mannit (direkt verpressbar) 78 mg
■ Hilfsstoffe (wie in Beispiel 1) 15 mg

Eigenschaften der pressfertigen Mischung und der Tabletten:
■ Kompressibilität und Fließfähigkeit: befriedigend bis gut
■ Mittlere Härte: 95 N
■ Abrieb: < 0,1 % (bestimmt nach Ph. Eur.)
■ Zerfallzeit: 75 Sek. (bestimmt nach Ph. Eur.)
■ Freisetzung: 84% Drospirenon nach 15 Min.
   92% Ethinylestradiol nach 15 Min.

Die so erhaltenen Tabletten können gegebenenfalls mit einem Überzug versehen werden.

### Beispiel 3: Herstellung von Tabletten mit Drospirenon, Rezeptur 3 (erfindungsgemäß)

Zu einer Lösung von 1 g Drospirenon in 20 ml Aceton/Isopropanol werden 4 g Polymethacrylat (Eudragit® E) gegeben und unter leichtem Erwärmen (30 bis 40°C) gelöst. Die klare Lösung wird gefriergetrocknet, so dass ein feines Wirkstoff-haltiges Pulver entsteht. Das Pulver wird mit pharmazeutischen Hilfsstoffen gemäß folgender Rezeptur vermischt und zu Tabletten verpresst:
■ Drospirenon-Pulver, entsprechend 3,0mg Drospirenon 15 mg
■ Lactose (direkt verpressbar) 85 mg
■ Hilfsstoffe (wie in Beispiel 1) 15 mg

Eigenschaften der pressfertigen Mischung und der Tabletten:
■ Kompressibilität und Fließfähigkeit: befriedigend bis gut
■ Mittlere Härte: 91 N
■ Abrieb: < 0, 1 % (bestimmt nach Ph. Eur.)
■ Zerfallzeit: 87 Sek. (bestimmt nach Ph. Eur.)
■ Freisetzung: 90% nach 15 Min.

Die so erhaltenen Tabletten können gegebenenfalls mit einem Überzug versehen werden.

## Patentansprüche

1. Verfahren zur Herstellung von Drospirenon-haltigen Wirkstoffpulvern, **dadurch gekennzeichnet, dass** man von einer Lösung des Drospirenons in wenigstens einem organischen Lösungsmittel, mit einem Gesamtwassergehalt des Lösungsmittels von nicht mehr als 15%, bevorzugt nicht mehr als 5%, ausgeht, ein pharmazeutisch akzeptables Adsorbermaterial, ausgewählt aus der Gruppe der Polymerisate auf Basis von Acryl- und/oder Methacrylsäure oder deren Aminoalkylestern oder deren Ammonioalkylestern und/oder deren Alkylestern, ganz oder teilweise darin löst oder dispergiert und das Lösungsmittel entfernt.

2. Verfahren nach Anspruch 1, **dadurch gekennzeichnet, dass** das Adsorbermaterial aus der Gruppe pharmazeutischer Polymethacrylate, die aus Methacrylsäure-Copolymeren, Aminoalkylmethacrylat-Copolymeren mit 1 bis 4 Kohlenstoffatomen im Alkylsubstituenten, Methacrylsäurester-Copolymeren, Ammonioalkylmethacrylat-Copolymeren mit 1 bis 4 Kohlenstoffatomen im Alkylsubstituenten besteht, ausgewählt ist.

3. Verfahren gemäß irgendeinem der Ansprüche 1 oder 2, **dadurch gekennzeichnet, dass** man ein Verhältnis von Wirkstoff zu Adsorbermaterial im Bereich von 1:0,05 bis 1:20, insbesondere im Bereich von 1:1 1 bis 1:10 einstellt.

4. Verfahren gemäß einem oder mehreren der vorhergehenden Ansprüche 1 bis 3, **dadurch gekennzeichnet, dass** man als Lösungsmittel organische Lösungsmittel mit einem Gesamtwasseranteil von nicht mehr als 15 Vol.-%, alleine oder in Mischungen einsetzt, wobei die organischen Lösungsmittel ausgewählt sind aus der Gruppe der niederen Alkanole mit 1 bis 4 Kohlenstoffatomen, der Gruppe der Ether, der Ester und der Gruppe der alipathischen Ketone und deren Gemischen.

5. Verfahren gemäß Anspruch 4, **dadurch gekennzeichnet, dass** man als Lösungsmittel ein Lösungsmittel aus der Gruppe, bestehend aus Methanol, Ethanol, Isopropanol, n-Propanol, Aceton, Ethylacetat, Methylethylketon, Methyl-t-butylester, Diethylether, Acetonitril, Tetrahydrofuran oder einem Gemisch daraus einsetzt, wobei das Lösungsmittel insbesondere unter Inertgasatmosphäre gehalten wird.

6. Drospirenon-haltige Wirkstoffpulver, enthaltend Drospirenon, gegebenenfalls in Kombination mit wenigstens einem weiteren pharmazeutisch-aktiven Wirkstoff, **dadurch gekennzeichnet, dass** das Drospirenon-haltige Wirkstoffpulver nach dem Verfahren gemäß einem oder mehreren der Ansprüche 1 bis 5 herstellbar ist.

7. Drospirenon-haltige Wirkstoffpulver, enthaltend Drospirenon gemäß Anspruch 7, **dadurch gekennzeichnet, dass** der weitere aktive Wirkstoff aus der Gruppe der Estrogene ausgewählt ist.

8. Pharmazeutische Formulierung mit mindestens einem aktiven Wirkstoff und gegebenenfalls weiteren pharmazeutisch akzeptablen Hilfs- und Trägerstoffen, **dadurch gekennzeichnet, dass** sie als Wirkstoff Drospirenon-haltiges Wirkstoffpulver gemäß einem oder mehreren der vorhergehenden Ansprüche 1 bis 7 enthält.

9. Pharmazeutische Formulierung gemäß Anspruch 8, **dadurch gekennzeichnet, dass** sie in Form von Tabletten und Granulaten vorliegt, die auf bekannte Weise mit üblichen pharmazeutisch akzeptablen Hilfsstoffen herstellbar sind.

10. Pharmazeutische Formulierung gemäß Anspruch 8 oder 9 in Form von den Wirkstoff schnell freisetzenden, durch Direktverpressung hergestellten Tabletten.

11. Pharmazeutische Formulierung gemäß einem der Ansprüche 8 bis 10, **dadurch gekennzeichnet, dass** der Anteil an Bindemittel in der Gesamtmischung der Formulierung 0 bis zu 20 Gew.-%, und der Anteil an Füll- und Hilfsstoffen in der Gesamtmischung der Formulierung 20 bis 99 Gew.-%, bevorzugt 50 bis 99 Gew.-%, beträgt.

12. Verwendung von Drospirenon-haltigen Wirkstoffpulvern, insbesondere herstellbar nach dem Verfahren gemäß irgendeinem der Ansprüche 1 bis 6, in morphologisch lagerstabiler Form in oralen pharmazeutischen Formulierungen.
